# EUROPEAN PATENT APPLICATION

(11) **EP 4 708 207 A1**
(43) Date of publication of application: **11.03.2026**
(21) Application number: 24199494.6
(22) Date of filing: 10.09.2024
(51) Int. Cl.: G06T 19/00, A61B 34/10, G16H 50/00

(54) **MEDICAL INTERVENTION PLANNING ASSISTANCE**

(71) Applicant: Siemens Healthineers AG, 91301 Forchheim (DE)
(72) Inventor: Petkov, Kaloian, Lawrenceville, 08648 (US); Shah, Rishabh, Wentworth Point, 2127 (AU)
(74) Representative: Siemens Healthineers Patent Attorneys

(57) **Abstract**

Medical imaging data (18) comprising a representation of an ROI including a segmented representation of a target object (8) is received. A surface of the target object (8) is determined and an SDF (19) comprising an orthogonal distance of the respective voxel from the surface of the target object (8), is generated. A rendered output representation (7) comprises:
i. a visualization (12) of a safety margin (9) surrounding the target object (8) generated based on the SDF (19); and/or
ii. a visualization (13) the target object (8) depending on distances of a resection surface from the surface of the target object (8), which are determined based on the SDF (19) map; and/or
iii. a visualization (14) of the resection surface, which depends on the distances of the resection surface from the surface of the at least one target object (8), which are determined based on the SDF (19) map.

## Description

The present invention is directed to a computer-implemented method for medical intervention planning assistance, to a data processing system for carrying out said computer-implemented method, to a medical imaging system comprising said data processing system, and to a respective computer program product.

In medical intervention planning, it is desirable to provide a visualization of the relevant objects in the region of interest, ROI, in the patient's body. For example, planning for liver procedures such as tumor ablation, resection or embolization benefits from a real-time visualization of the tumor to be treated as well as vessel subtrees feeding the tumor et cetera.

Existing techniques use simple geometric shapes, such as sphere proxies, for the tumor and, for example, for a safety margin in embolization planning or low-order Bezier surfaces in resection planning. Feeding vessels are typically visualized using line graphics or extruded cylinders if a vessel branch diameter is available.

For more complicated tumor surfaces or more complex situations of multiple objects to be considered, for example, the accuracy of said methods is limited.

It is an objective of the present invention to provide an improved concept for medical intervention planning assistance, which increases the accuracy of the information provided to the person or algorithm carrying out the planning.

This objective is achieved by the subject-matter of the independent claim. Further implementations and preferred embodiments are subject-matter of the dependent claims.

The improved concept is based on the idea to determine a signed distance field, SDF, with respect to the surface of at least one target object and to use the SDF during the rendering of an output representation for the medical intervention planning assistance.

According to an aspect of the invention, a computer-implemented method for medical intervention planning assistance or, in other words, for assisting the planning of the medical intervention is provided. Therein, medical imaging data comprising a three-dimensional representation of a region of interest, ROI, is received. The medical imaging data or, in particular, the three-dimensional representation of the ROI, includes a segmented three-dimensional representation of at least one target object in the ROI. A surface of the at least one target object is determined based on the medical imaging data and a signed distance field, SDF, is generated, wherein the SDF comprises, for each voxel of a plurality of voxels of the three-dimensional representation of the ROI, an orthogonal distance of the respective voxel from the surface of the at least one target object. An output representation for the medical intervention planning assistance is rendered based on the medical imaging data. The rendered output representation comprises:
i. a visualization of a safety margin surrounding the at least one target object at a predefined safety distance from the surface of the at least one target object, wherein the visualization of the safety margin is generated based on the SDF, and/or
ii. a visualization of the at least one target object, in particular the surface of the at least one target object, wherein the visualization depends on first distances of the surface of the at least one target object from a predetermined resection surface. The first distances are determined based on the SDF, and/or
iii. a visualization of the predetermined resection surface, wherein the visualization of the predetermined resection surface depends on second distances of the predetermined resection surface from the surface of the at least one target object. The second distances are determined based on the SDF.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for medical intervention planning assistance, which is not purely computer-implemented, is obtained by including respective steps of generating the medical imaging data, for example by a CT-device or a CBCT-device or an MRI-device.

Furthermore, further embodiments of the computer-implemented method or the method for medical intervention planning assistance are obtained by including the step of generating the segmented three-dimensional representation of at least one target object based on the three-dimensional representation of the ROI. In particular, instead of receiving the medical imaging data, the three-dimensional representation of the ROI is received and the segmented three-dimensional representation of at least one target object is generated based on the three-dimensional representation of the ROI.

In medical intervention planning, it is, for example, planned by a human manually or by an algorithm automatically or semi-automatically, where in the ROI certain cuts shall be placed or medical devices shall be applied et cetera. By generating the rendered output representation, the person or algorithm planning the medical intervention is assisted, since the rendered output representation provides a comprehensive overview of the relevant objects in the ROI. In particular, the rendered output representation comprises a respective visualization of all relevant objects including, in particular, the at least one target object in the ROI. The ROI is, in particular, a three-dimensional region in a patient's body.

In particular, assistance information including or consisting of the rendered output representation may be stored to a storage device for further usage and/or displayed on a display device, in particular to assist the person or algorithm at the medical intervention planning.

In particular, the medical intervention may be a tumor embolization intervention, wherein the feeding vessels of the tumor are clogged with particles or another material or are dissected. The at least one target objects comprise the tumor in this case. In this case, the variant of the computer-implemented method according to i) is particularly beneficial, since the safety margin provides a valuable guidance to the person or algorithm planning the medical intervention in the sense that it is simplified to find the positions wherein the feeding vessels can safely be dissected or accessed without risking any unwanted damage.

The medical intervention may also be, for example, a resection of a tumor or another object from the ROI. The at least one target objects comprise the tumor or other object to be resected in this case. In this case, the variants of the computer-implemented method according to ii) and iii) are particularly beneficial, since, by visualizing the at least one target object or the resection surface depending on the distances from the surface of the at least one target object, the person or algorithm planning the medical intervention can effectively and comprehensively be assisted in the sense that the safe placement of the resection surface without risking unwanted damage by the resection is simplified.

The at least one target object may consist of one target object, for example the tumor. The at least one target object may also comprise two or more target objects which may or may not be in physical contact with each other. The surface of the at least one target object can, for example, be understood as a union of all individual surfaces of the target objects. Consequently, the surface of the at least one target object is not necessarily a single connected surface.

The three-dimensional representation of the ROI may, for example, be given by a computed tomography, CT, reconstruction representing the ROI and/or a three-dimensional cone beam CT, CBCT, reconstruction representing the MRI and/or a three-dimensional magnetic resonance imaging, MRI, reconstruction representing the ROI.

For example, the three-dimensional representation of the ROI may be given in terms of a plurality of voxels of the ROI and respective image values for each of the voxels. The segmented three-dimensional representation of the at least one target object may, for example, be a binary representation, which may, for example, assign a first value, for example zero, to any voxel, which does not belong to the at least one target object, and a second value, for example one, to any voxel, which belongs completely or partly to the at least one target object. The segmented three-dimensional representation of the at least one target object may be provided in addition to the three-dimensional representation of the ROI, for example, as an additional set of the plurality of voxels with said binary values instead of the image value as explained. It is also possible to provide the segmented three-dimensional representation of the at least one target object as a part of the three-dimensional representation of the ROI, for example as a further channel of the three-dimensional imaging data and so forth.

The signed distance field, SDF, which may also be denoted as signed distance function or signed distance field may, for example, has a value of zero for points on the surface of the at least one target object and a non-zero value at all other points in the ROI. A common convention is, in particular for one or more closed surfaces as reference for the SDF, to assign a positive value to points outside of the surface of the at least one target object and a negative value to points inside the surface of the at least one target object. However, also the opposite convention is possible. The values of the SDF correspond to the orthogonal distance of the respective point to the surface of the at least one target object.

The SDF can be realized and stored in different ways. For example, the SDF may correspond to a plurality of voxels in the ROI as described for the three-dimensional representation of the ROI contained by the medical imaging data. In particular, each voxel of the ROI may have a correspondingly assigned distance value of the SDF as described. It is also possible to have a higher resolution of the SDF compared to the plurality of voxels of the three-dimensional representation of the ROI. In some cases, it is also possible to have a continuous definition of the SDF or a definition of the SDF on a mesh in the ROI.

In case the distance value of the SDF is a distance between two voxels of the plurality of voxels in the ROI, the distance may, for example, be defined with respect to the corresponding center point of the voxels or other characteristic points of the voxels, for example, their corner points, et cetera. It is possible, however not mandatory, that the distance value of the SDF is computed with respect to the voxels of the plurality of voxels forming the surface of the at least one target object. Rather, it is also possible that another approximation or representation of the surface of the at least one target object, for example in form of a mesh, is computed and the distances of the SDF are determined based on the approximation or representation of the surface, for example the mesh.

The rendering itself may be carried out according to a conventional method for three-dimensional image rendering to represent the content of the medical imaging data in the ROI, including, in particular, the at least one target object or its surface, respectively. To this end, predefined model parameters may be provided in the output representation and be rendered depending on the medical imaging data and the predefined model parameters or rendering model parameters.

In contrast to conventional methods, however, the SDF is exploited in all variants of the computer-implemented method i), ii), iii) to determine the distances of certain objects or virtual objects like the resection surface or the safety margin from the surface of the at least one target object.

The safety margin is defined as a surface, which has a constant orthogonal distance from the surface of the at least one target object. Consequently, the SDF is particularly suitable for a fast and simple evaluation of the distances and specification of the safety margin and can therefore be used in real-time during the rendering process for visualizing the safety margin. The safety margin may be a single closed surface or may consist of several surfaces, depending on the position, size and number of the at least one target object.

In the variant ii), the at least one target object, for example its surface, is visualized in the rendered output representation depending on the first distances of the surface of the at least one target object from the predetermined resection surface. The first distances may also be easily determined from the SDF in real-time during the rendering process. The first distances correspond to orthogonal distances of all or multiple points on the at least one target object from the resection surface. For example, a color or shading or structure or another property of the visualization of the at least one target object may be depending on the respective first distance.

In the variant iii), the resection surface is visualized in the rendered output representation depending on the second distances of the predetermined resection surface from the surface of the at least one target object. These distances may also be easily determined from the SDF in real-time during the rendering process. The second distances correspond to orthogonal distances of all or multiple points on the resection surface from the surface of the at least one target object. For example, a color or shading or structure or another property of the visualization of the resection surface may be depending on the respective second distance.

Using the SDF for the visualizations in the rendered output representation has several advantages including, for example, their simple and fast processability at runtime during the rendering. In particular, when rendering safety margins, SDF allows rendering of the surface at any arbitrary distance from the encoded SDF surface. That is, different safety margin distances can be rendered from the same SDF without having to load separate datasets representing specific distance values. Furthermore, using the SDF also allows for visualization of multiple complex objects or surfaces in the ROI or virtual surfaces like the safety margin et cetera. Also, for complex structures or shapes or compositions of the at least one target object, the SDF allows for particularly accurate representations. The rendered output representation is, in particular, displayed on a display device, in particular a display device of the data processing system, and/or stored to a storage device, in particular a storage device of the data processing system.

A further advantage is the possibility to include various interactions with the user during the rendering process, for example to fine-tune the safety margin or visualizations of the rendered output representation.

According to several embodiments, the SDF is evaluated, in particular at runtime, during the rendering in order to generate the visualization of the safety margin and/or to generate the visualization of the at least one target object and/or to generate the visualization of the predetermined resection surface.

Consequently, the required computational resources and, in particular, computational time may be reduced.

According to several embodiments, the surface of the at least one target object is determined as a subset of the plurality of voxels depending on the segmented three-dimensional representation of at least one target object. The orthogonal distance for a respective voxel of the plurality of voxels is computed depending on a position of the respective voxel, for example its center position or corner position, and the respective position, for example its center position or a corner position, of a voxel of the subset of the plurality of voxels.

In other words, the SDF is determined on voxel level. In yet other words, it is not required to further process or model the surface of the at least one target object in order to generate the SDF, which saves computational time and further computational resources.

According to at least one embodiment, a preliminary surface of the at least one target object is determined as a subset of the plurality of voxels depending on the segmented three-dimensional representation of the at least one target object. The surface of the at least one target object is determined as a surface mesh depending on the preliminary surface of at least one target object. The orthogonal distance for a respective voxel of the plurality of voxels is computed depending on a position of the respective voxel, in particular its center position or a corner position etc. and depending on the mesh or in other words as an orthogonal distance of the position of the respective voxel from the surface mesh.

The surface mesh is, in particular, a polygonal mesh, for example a triangular mesh or a rectangular mesh.

As described with respect to the surface of the at least one target object in the previous embodiments, the preliminary surface corresponds to the subset of the plurality of voxels in the present embodiments. However, in contrast to the previous embodiments, the surface according to which the SDF is finally computed is given by the surface mesh. The mesh models or approximates the preliminary surface and, for example, interpolates between the voxels. Consequently, the surface is smoother in such embodiments and, consequently, the SDF may be determined with higher accuracy.

According to several embodiments, the rendered output representation comprises the visualization of the safety margin, and the predefined safety distance lies in the range [10 mm, 120 mm] or in the range [20 mm, 100 mm].

According to several embodiments, the rendered output representation comprises the visualization of the safety margin, and the rendered output representation comprises a visualization of a first further object within the ROI, wherein the visualization of the first further object depends on whether the first further object lies inside the safety margin or not.

The first further object may be part of an organ structure, for example, of a vessel structure, or the like. In such embodiment, different visualizations of different parts of said organ structure are possible, depending on whether the respective parts lie inside the safety margin or outside the safety margin.

In such embodiments, the person or algorithm planning the medical intervention may be further assisted by indicating directly via the visualization whether the respective first further object lies inside the safety margin should therefore not be cut, for example, or not.

It is noted that it is determined based on the SDF whether or not the first further object lies inside the safety margin.

According to several embodiments, the rendered output representation comprises a visualization of a second further object within the ROI, wherein the visualization of the second further object depends on a distance of the second further object from the surface of the at least one target object, wherein said distance is determined based on the SDF.

The second further object may be, for example, parenchyma, for example liver parenchyma, vessels or other objects in the ROI. For example, liver lobe boundaries, in particular represented by a segmentation of the liver parenchyma, are an example of an object that may be visualized together with the safety margin. For example, the color the liver lobes that fall within the safety margin may be chosen differently from other liver lobes based on the SDF values.

For example, the color or surface structure et cetera of the second further object may depend on how large the respective distance from the at least one target object is. In this way, the method may further assist the person or algorithm planning the medical intervention.

According to several embodiments, the medical imaging data or the three-dimensional representation of the ROI includes a segmented three-dimensional representation of at least one further target object.

Definitions and explanations above regarding the segmented three-dimensional representation of the at least one target object hold analogously for the segmented three-dimensional representation of the at least one further target object.

The at least one further target object may, for example, correspond to one or more vessels, in particular blood vessels feeding the at least one target object. In particular, the at least one target object comprises a tumor, which is fed by the feeding vessels.

In particular, the rendered output representation comprises a visualization of the at least one further target object.

According to several embodiments, a surface of the at least one further target object is determined based on the medical imaging data, and a further SDF is generated, wherein the further SDF comprises, for each voxel of the plurality of voxels of the three-dimensional representation of the ROI, a further orthogonal distance of the respective voxel from the surface of the at least one further target object.

The rendered output representation comprises a visualization of the at least one target object, in particular the surface of the at least one target object, wherein said visualization depends on third distances of the surface of the at least one target object from the surface of the at least one further target object, wherein said distances are determined based on the further SDF.

Alternatively or in addition, the visualization of the safety margin depends on fourth distances of the safety margin from the surface of the at least one further target object, wherein said fourth distances are determined based on the further SDF.

For example, a heat-map which indicates the third distances or the fourth distances, respectively, may be rendered on the surface of the at least one target object or on the surface of the safety margin, respectively.

In this way, it is pointed out more directly, how far the at least one further target object, for example feeding vessels, lie away from the at least one target object, for example the tumor, and/or the safety margin.

According to several embodiments, the output representation comprises a visualization of the at least one further target object, in particular the surface of the at least one further target object, wherein said visualization depends on fifth distances of the surface of the at least one further target object from the surface of the at least one target object, wherein the fifth distances are determined based on the SDF map.

Consequently, also the distances of the at least one further target object from the surface of the at least one target object is directly representing the visualization of the at least one further target object, which improves the assistance of the medical intervention planning further.

According to several embodiments, the predetermined resection surface is received as a constructive solid geometry, CSG, surface.

This is particularly beneficial, since the CSG surfaces have many properties in common with SDF maps or surfaces in SDF representations. Therefore, the visualization of the at least one target object depending on the first distance and/or the visualization of the resection surface depending on the second distance is simplified.

According to a further aspect of the invention, a data processing system is provided, which is adapted to perform a computer-implemented method according to the invention.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a medical imaging system is provided. The medical imaging system comprises a data processing system according to the invention and a medical imaging device. The medical imaging device is configured to generate imaging raw data representing the ROI, and the data processing system is configured to generate the medical imaging data depending on the imaging raw data.

The medical imaging data may, for example, be a CT device, a CBCT device, an MRI device et cetera.

Further implementations of the medical imaging system according to the invention follow directly from the various embodiments of the computer implemented method according to the invention and vice versa. In particular, individual features and corresponding explanations as well as advantages relating to the various implementations of the computer implemented method according to the invention can be transferred analogously to corresponding implementations of the medical imaging system according to the invention. In particular, the medical imaging system according to the invention is designed or programmed to carry out the computer implemented method according to the invention. In particular, the medical imaging system according to the invention carries out the computer implemented method according to the invention.

According to a further aspect of the invention, a computer program product comprising instructions is provided. When the instructions are executed by a data processing system, the instructions cause the data processing system to carry out a computer-implemented method according to the invention.

The instructions may be provided as program code, for example. The program code can for example be provided as binary code or assembler and/or as source code of a programming language, for example C, and/or as program script, for example Python.

According to a further aspect of the invention, a computer-readable storage medium, in particular a tangible and/or non-transient computer readable storage medium, storing a computer program according to the invention is provided.

The computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the Figures,
- FIG 1: shows schematically an exemplary embodiment of a medical imaging system according to the invention;
- FIG 2: shows a schematic flow diagram of an exemplary embodiment of a computer-implemented method for medical intervention planning assistance according to the invention; and
- FIG 3: shows a schematic flow diagram of a further exemplary embodiment of a computer-implemented method for medical intervention planning assistance according to the invention.

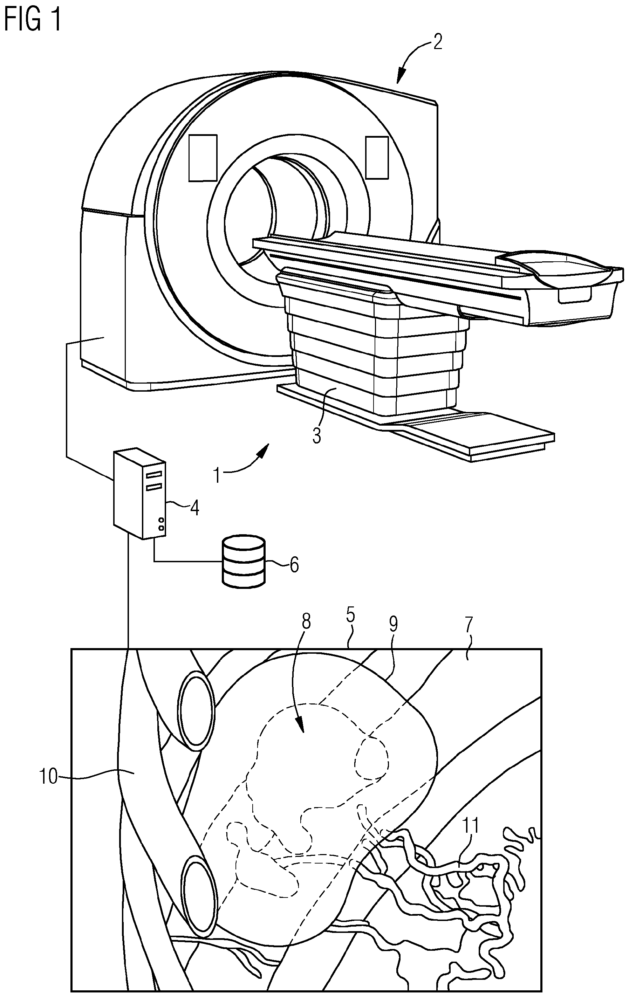

FIG 1 shows schematically an exemplary embodiment of a medical imaging system 1 according to the invention. The medical imaging system 1 comprises a data processing system 4, 5, 6 according to the invention, which adapted to carry out a computer-implemented method for medical intervention planning assistance according to the invention, as well as a medical imaging device 2, which may for example be a CT-device. Optionally, the medical imaging system 1 comprises a patient table 3 or the like. The medical imaging device 2 is configured to generate imaging raw data representing a region of interest, ROI, in a patient's body, and the data processing system 4, 5, 6 is configured to generate medical imaging data 18 depending on the imaging raw data. The medical imaging data 18 comprises a three-dimensional representation of the ROI, including a segmented three-dimensional representation of at least one target object 8, for example one or more tumors.

A result of the computer-implemented method for medical intervention planning assistance according to the invention comprises a rendered output representation 7 for the medical intervention planning assistance, which, for example, shows at least the content in the ROI comprised by the medical imaging data, including the at least one target object 8 and, for example other objects like vessels 11, in particular feeding vessels of the one or more tumors, bones 10, et cetera. The rendered output representation 7 may also show one or more virtual objects, for example resection surfaces or a safety margin 9 surrounding the at least one target object 8 at a predefined distance. The rendered output representation 7 may be displayed on a display device 5 of the data processing system 4, 5, 6 and/or stored to a storage device 6 of the data processing system 4, 5, 6.

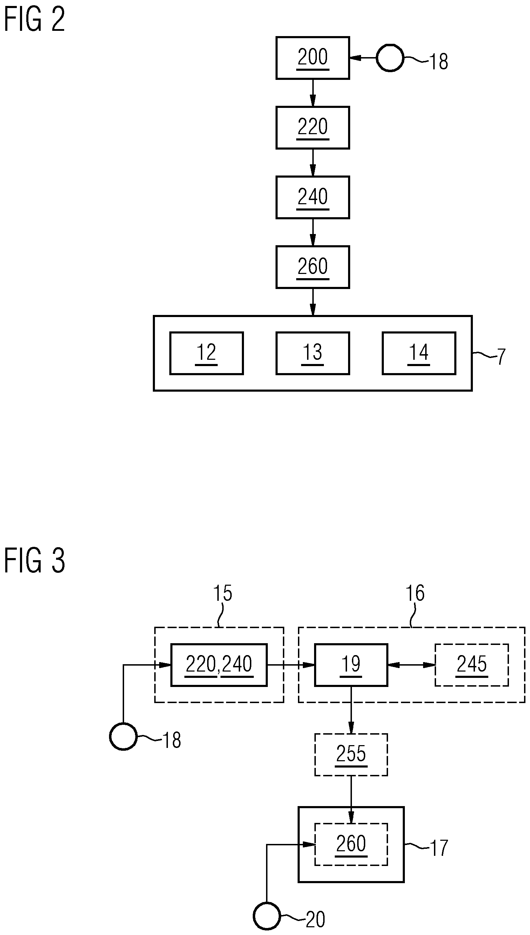

FIG 2 shows a schematic flow diagram of an exemplary embodiment of a computer-implemented method for medical intervention planning assistance according to the invention, which may for example be carried out by the data processing system 4, 5, 6 of FIG 1.

In step 200, the medical imaging data 18 as described above are received. In step 220, a surface of the at least one target object 8 is determined based on the medical imaging data 18 and in step 240, a signed distance field, SDF, 19 (see FIG 3) is generated, wherein the SDF 19 comprises, for each voxel of a plurality of voxels of the three-dimensional representation of the ROI, an orthogonal distance of the respective voxel from the surface of the at least one target object 8. In step 260, the output representation 7 for the medical intervention planning assistance is rendered, in particular based on predetermined rendering model parameters 20 (see FIG 3), based on the medical imaging data 18.

The rendered output representation 7 may comprise a visualization 12 of the safety margin 9 surrounding the at least one target object 8 at a predefined safety distance from the surface of the at least one target object 8. Therein, the visualization 12 of the safety margin 9 is generated based on the SDF 19.

Alternatively or in addition, the rendered output representation 7 may comprise a visualization 13 of the at least one target object 8, which depends on first distances of the surface of the at least one target object 8 from a predetermined resection surface. The first distances are determined based on the SDF 19.

Alternatively or in addition, the rendered output representation 7 may comprise a visualization 14 of the predetermined resection surface, which depends on second distances of the predetermined resection surface from the surface of the at least one target object 8. The second distances are determined based on the SDF 19.

FIG 3 shows a schematic flow diagram of a further exemplary embodiment of a computer-implemented method for medical intervention planning assistance according to the invention, which is based on FIG 2. Therein, the different method steps are associated to a preprocessing phase 15, a real-time editing phase 16, and a real-time rendering phase 17. In particular, the method steps 220 and 240 of FIG 2 may be carried out during the preprocessing phase 15, and the method step 260 may be carried out during the real-time rendering phase 17.

In some embodiments, SDF editing is done in method step 245 during the real time editing phase 16 and/or SDF operations may be performed in method step 255 during the real time editing phase 16 or during the real-time rendering phase 17 or between them.

In some embodiments, the medical intervention to be planned corresponds to an embolization intervention, for example a tumor embolization. In such embodiments, the visualization of the safety margin 9 as described is particularly beneficial. In particular, since the SDF 19 encodes the distance from the surface of the at least one target object 8, the tumor in this case, the rendering engine may visualize the safety margin 19 at any predefined safety distance in real-time without additional processing.

In some embodiments, during the SDF editing step, the SDF 19 is further refined, for example manually using tools based on SDF operations, and the refinements may be visualized in real-time.

In some embodiments, other structures, like vessels 11, are shaded based on their distance from the at least one target object 8, or based on whether they fall within the safety margin 19.

In some embodiments, a further SDF relative to the vessels' 11 surface is used to render a heatmap on the surface of the at least one target object 8 and/or on the safety margin 19 to highlight the parts of the surface closer to the feeding vessels 11. This may be done in the SDF operations step.

In a representative example, the rendering engine may perform the following steps during the volume raycasting to incorporate the SDFs for visualizing the vessel 11 and safety margin 19 intersection (pseudo code):

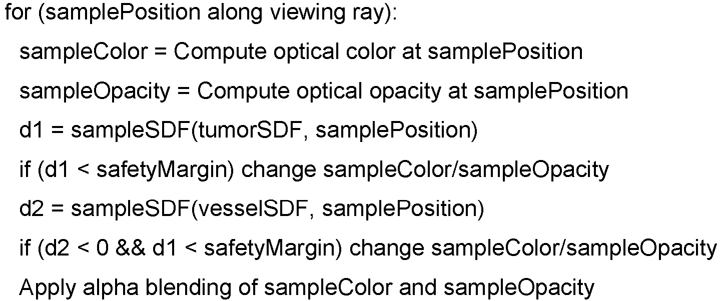

In some embodiments, individual SDFs of vessel segments may computed from automated segmentation as a pre-process. These segments may be grouped into vessel subtrees at runtime. Liver parenchyma may for example be partitioned based on distances to these subtrees in the same step. This may be done in the SDF operations step and may be achieved in a single compute shader pass before the rendering. The vessel segments, subtrees and parenchyma partitions may further be edited manually in real-time using SDF operations. As SDFs are continuous surface representations, the vessel subtrees and partitioned liver parenchyma are also continuous and do not exhibit quantization artifacts like staircase for volumes and finite resolution for meshes.

In some embodiments, the medical intervention to be planned corresponds to a resection intervention, for example a tumor resection. In such embodiments, the visualization of the resection surface or the at least one target object 8 depending on the distances from the resection surfaces, as described is particularly beneficial. The resection surface may be provided as a CSG surface, for example.

In some embodiments, respective CSG representations of resection surfaces are used to simulate the impact of resection cuts on liver parenchyma, the tumor and/or its feeding vessels represented by SDFs. The CSG surfaces and their interactions with the SDFs may be manipulated and the SDFs may be edited in, for example in the SDF operations step, with immediate visual feedback using real-time raycasting of the SDF.

In some embodiments, an SDF representation of the resection surface allows the distance to be projected onto the vessels, tumor, liver segments et cetera, to anticipate error margins and risks from the planned cuts for example in the SDF operations step.

As a representative example, the rendering engine performs the following steps during the volume raycasting to visualize the distance to the resection surface onto the vessels and the vascular territory for a vessel subtree (pseudo code):

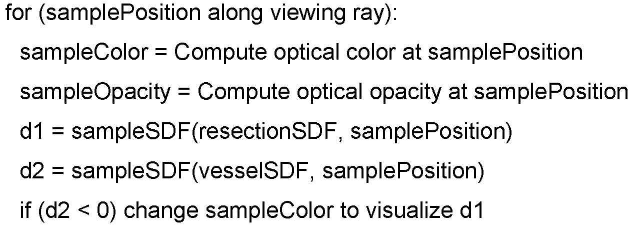

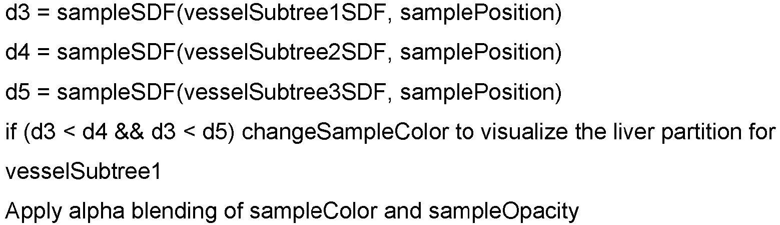

As the SDF 19 is a continuous field, the surface may be rendered with effects that cannot be applied to conventional meshes. By using the SDF 19, the surfaces may be rendered with any thickness to achieve the desired visual effect. The distance values in the SDF may be used to apply volumetric effects to rendering, like a fog effect inside safety margin changing with proximity to the at least one target object. Such effects may for example be defined by the rendering modelling parameters 20 and do not require changes to the SDF 19 itself.

In some embodiments, the SDF 19, once computed as described, may be converted to a mesh, which can then be rendered as such using derivatives of the Marching Cubes algorithm, for example, including neural and real-time variants. This is advantageous in solutions requiring low memory footprint and on hardware capable of mesh ray tracing.

Using the SDF has significant advantages compared to using simple geometric primitives or pre-segmented meshes to visualize safety margins or tumors. While meshes have a limited resolution, the continuous representation with SDFs results in higher quality images and allows for visualization of distance information during the rendering. For determining the intersections of vessels, safety margins and resection surface, mesh representations require complex geometry processing and explicit modeling of the intersection graphical primitives. In contrast, SDFs may model the intersection implicitly via simple mathematical operations on the distance field during rendering. As SDF represent not just a surface but a field of distances from a surface, the safety margin distance can be altered at runtime directly during rendering without having to alter the dataset and with immediate visual feedback of the distances to clinically relevant structures.

Independent of the grammatical term usage, individuals with male, female or other gender identities are included within the term.

## Claims

1. Computer-implemented method for medical intervention planning assistance, wherein
- medical imaging data (18) comprising a three-dimensional representation of a region of interest, ROI, including a segmented three-dimensional representation of at least one target object (8) is received;
- a surface of the at least one target object (8) is determined based on the medical imaging data (18) and a signed distance field, SDF, (19) is generated, wherein the SDF (19) comprises, for each voxel of a plurality of voxels of the three-dimensional representation of the ROI, an orthogonal distance of the respective voxel from the surface of the at least one target object (8);
- an output representation for the medical intervention planning assistance is rendered based on the medical imaging data (18); and
- the rendered output representation (7) comprises:
i. a visualization (12) of a safety margin (9) surrounding the at least one target object (8) at a predefined safety distance from the surface of the at least one target object (8), wherein the visualization (12) of the safety margin (9) is generated based on the SDF (19); and/or
ii. a visualization (13) the at least one target object (8), which depends on first distances of the surface of the at least one target object (8) from a predetermined resection surface, which are determined based on the SDF (19); and/or
iii. a visualization (14) of the predetermined resection surface, which depends on second distances of the predetermined resection surface from the surface of the at least one target object (8), which are determined based on the SDF (19).

2. Computer-implemented method according to claim 1, wherein the SDF (19) is evaluated during the rendering in order to generate the visualization (12) of the safety margin (9) and/or to generate the visualization (13) of the at least one target object (8) and/or to generate the visualization (14) of the predetermined resection surface.

3. Computer-implemented method according to one of the preceding claims, wherein the medical imaging data (18) comprises a three-dimensional computed tomography, CT, reconstruction representing the ROI and/or a three-dimensional cone-beam CT reconstruction representing the ROI and/or a three-dimensional magnetic resonance imaging reconstruction representing the ROI.

4. Computer-implemented method according to one of the preceding claims, wherein
- the surface of the at least one target object (8) is determined as a subset of the plurality of voxels depending on the segmented three-dimensional representation of at least one target object (8); and
- the orthogonal distance for a respective voxel of the plurality of voxels is computed depending on a position of the respective voxel and a respective position of a voxel of the subset of the plurality of voxels.

5. Computer-implemented method according to one of claims 1 to 3, wherein
- a preliminary surface of the at least one target object (8) is determined as a subset of the plurality of voxels depending on the segmented three-dimensional representation of the at least one target object (8);
- the surface of the at least one target object (8) is determined as a surface mesh depending on the preliminary surface of at least one target object (8); and
- the orthogonal distance for a respective voxel of the plurality of voxels is computed depending on a position of the respective voxel and depending on the surface mesh.

6. Computer-implemented method according to one of the preceding claims, wherein
- the rendered output representation (7) comprises the visualization (12) of the safety margin (9); and
- the predefined safety distance from the surface of the at least one target object (8) lies in the range [10 mm, 120 mm] or in the range [20 mm, 100 mm].

7. Computer-implemented method according to one of the preceding claims, wherein
- the rendered output representation (7) comprises the visualization (12) of the safety margin (9);
- and the rendered output representation (7) comprises a visualization of a first further object within the ROI, wherein the visualization of the first further object depends on whether the first further object lies inside the safety margin (9).

8. Computer-implemented method according to one of the preceding claims, wherein the rendered output representation (7) comprises a visualization of a second further object within the ROI, wherein the visualization of the second further object depends on a distance of the second further object from the surface of the at least one target object (8), which is determined based on the SDF (19).

9. Computer-implemented method according to one of the preceding claims, wherein the three-dimensional representation of the ROI includes a segmented three-dimensional representation of at least one further target object (11).

10. Computer-implemented method according to claim 9, wherein a surface of the at least one further target object (11) is determined based on the medical imaging data (18) and a further SDF (19) is generated, wherein the further SDF (19) comprises, for each voxel of the plurality of voxels of the three-dimensional representation of the ROI, a further orthogonal distance of the respective voxel from the surface of the at least one further target object (11), and
- the rendered output representation (7) comprises a visualization of the at least one target object (8), which depends on third distances of the surface of the at least one target object (8) from the surface of the at least one further target object (11), which are determined based on the further SDF (19); and/or
- the visualization (12) of the safety margin (9) depends on fourth distances of the safety margin (9) from the surface of the at least one further target object (11), which are determined based on the further SDF (19).

11. Computer-implemented method according to one of claims 9 or 10, wherein the rendered output representation (7) comprises a visualization of the at least one further target object (11), which depends on fifth distances of the surface of the at least one further target object (11) from the surface of the at least one target object (8), which are determined based on the SDF (19) map.

12. Computer-implemented method according to one of the preceding claims, wherein the predetermined resection surface is received as a constructive solid geometry surface.

13. Data processing system (4, 5, 6) adapted to carry out a computer-implemented method according to one of the preceding claims.

14. Medical imaging system (1) comprising a data processing system (4, 5, 6) according to claim 13 and a medical imaging device (2), which is configured to generate imaging raw data representing the ROI, wherein the data processing system (4, 5, 6) is configured to generate the medical imaging data (18) depending on the imaging raw data.

15. Computer program product comprising instructions, which, when executed by a data processing system (4, 5, 6), cause the data processing system (4, 5, 6) to carry out a computer-implemented method according to one of claims 1 to 12.
